# EUROPEAN PATENT APPLICATION

(11) **EP 4 821 598 A1**
(43) Date of publication of application: **14.10.2026**
(21) Application number: 24899245.5
(22) Date of filing: 10.07.2024
(51) Int. Cl.: B01L 3/00, C12Q 1/6806

(54) **MICROFLUIDIC SYSTEM FOR TARGET OBJECT PAIRING, AND PAIRING METHOD**

(30) Priority: 07.12.2023 CN 202311675778
(71) Applicant: Guangzhou National Laboratory, Guangzhou, Guangdong 510005 (CN)
(72) Inventor: CAO, Xiaobao, Guangzhou, Guangdong 510005 (CN); XU, Wenhua, Guangzhou, Guangdong 510005 (CN); JIN, Jian, Guangzhou, Guangdong 510005 (CN); ZENG, Lingliao, Guangzhou, Guangdong 510005 (CN); WANG, Zhong, Guangzhou, Guangdong 510005 (CN); LIU, Ya, Guangzhou, Guangdong 510005 (CN); HAN, Mengying, Guangzhou, Guangdong 510005 (CN); LI, Mengqi, Guangzhou, Guangdong 510005 (CN); XU, Tao, Guangzhou, Guangdong 510005 (CN)
(74) Representative: Torner, Juncosa I Associats, SL
(86) International application number: PCT/CN2024/104835
(87) International publication number: WO 2025/118594

(57) **Abstract**

The present disclosure provides a microfluidic system for target object pairing and a pairing method. The microfluidic system includes a first channel, a second channel, an oil channel, a buffer channel, a pairing channel, a pairing microvalve, a first microvalve, a second microvalve, and a buffer microvalve. The pairing channel is in communication with the oil channel. The first channel, the second channel, and the buffer channel are all in communication with the pairing channel. When a single first target and a single second target are present in the pairing channel, the first microvalve and the second microvalve are in a closed state, and the pairing microvalve and the buffer microvalve are in an open state, allowing a buffer in the buffer channel to push the single first target and the single second target in the pairing channel into oil phase in the oil channel to form a droplet. The present disclosure can prevent other first targets and/or other second targets from entering the droplet, thereby improving the success rate of encapsulation and reducing cell loss caused by co-encapsulation.

## Description

### TECHNICAL FIELD

The present disclosure relates to the field of single-cell sequencing technologies, and specifically to a microfluidic system for target object pairing and a pairing method.

### BACKGROUND

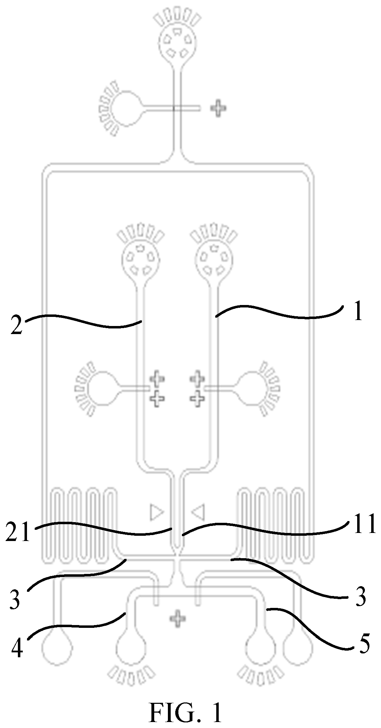

Single-cell RNA sequencing technology (scRNA-seq) reveals the heterogeneity of cells within tissues. As compared with traditional gene sequencing technologies, the single-cell sequencing technology has the advantages of high accuracy and high resolution, and can provide information at the single cell level and reveal the heterogeneity and complexity of expression in tissues. Therefore, the single-cell sequencing technology has been widely used in the fields of cancer, reproduction, immunity, and development. FIG. 1 shows a pairing system for pairing a single first target with a single second target, including a first channel 1, a second channel 2, an oil channel 3, a waste channel 4, a sample channel 5, and a plurality of microvalves corresponding to each channel. The microvalves can control the opening and closing of the respective channels. The first channel 1 includes a first stop region 11. The second channel 2 includes a second stop region 21. In use, a first liquid containing first targets is injected into the first channel 1, and a second liquid containing second targets is injected into the second channel 2. If no single first target appears in the first stop region 11, the first liquid is discharged through the waste channel 4. If no single second target appears in the second stop region 21, the second liquid is discharged through the waste channel 4, accordingly. If a single first target appears in the first stop region 11, a flow of the first liquid in the first channel 1 is blocked by a respective microvalve so that the single first target stops in the first stop region 11. If a single second target appears in the second stop region 21, the flow of the second liquid in the second channel 2 is blocked by a respective microvalve so that the single second target stops in the second stop region 21, accordingly. After both a single first target and a single second target stop at corresponding stop positions, the waste channel 4 is closed, and the oil channel 3 and the sample channel 5 are opened. Then, the first channel 1 and the second channel 2 are opened, so that the single first target and the single second target are respectively fed into an oil through the first liquid and the second liquid to form droplets, which then flow out through the sample channel 5. The above scheme has the following problems.
1. The power sources for propelling the single first target and the single second target into the oil are the first liquid and the second liquid, respectively. Therefore, the following problems may arise: (1). when the first liquid propels the single first target, a subsequent first target will also be encapsulated in the droplet; (2). when the second liquid propels the single second target, a subsequent second target will also be encapsulated in the droplet; (3). when the first liquid propels the single first target and the second liquid propels the single second target, the subsequent first and second targets will be encapsulated in the droplet together. As a result, the formed droplet contains multiple first targets and/or multiple second targets, leading to co-encapsulation that affects subsequent detection.
2. The scheme shown in FIG. 1 generates a large number of empty droplets, which need to be sorted from normal droplets to avoid contamination, resulting in slow processing and low efficiency.

### SUMMARY

The present disclosure aims to resolve at least one of the technical problems in the related art. Therefore, the present disclosure provides a microfluidic system for target object pairing.

The present disclosure also provides a pairing method.

A first embodiment of the present disclosure provides a microfluidic system for target object pairing, including a first channel, a second channel, an oil channel, a buffer channel, and a pairing channel. The pairing channel is in communication with the oil channel, and the first channel, the second channel, and the buffer channel are all in communication with the pairing channel.

The microfluidic system further includes a pairing microvalve corresponding to the pairing channel, a first microvalve corresponding to the first channel, a second microvalve corresponding to the second channel, and a buffer microvalve corresponding to the buffer channel. The pairing microvalve is configured for controlling opening and closing of the pairing channel. The first microvalve is configured for controlling opening and closing of the first channel. The second microvalve is configured for controlling opening and closing of the second channel. The buffer microvalve is configured for controlling opening and closing of the buffer channel.

When both the single first target and the single second target are present in the pairing channel, the first microvalve and the second microvalve are in a closed state, and the pairing microvalve and the buffer microvalve are in an open state, allowing a buffer solution in the buffer channel to push the single first target and the single second target in the pairing channel into an oil phase in the oil channel to form a droplet.

The microfluidic system for target object pairing according to the embodiment of the present disclosure at least has the following beneficial effects.

In this embodiment, a separate buffer channel is configured to utilize the buffer solution to propel the single first target A and the single second target B into the oil. This obviates the need to propel the single first target A and the single second target B through the first liquid and the second liquid, so that the first channel and the second channel can be kept in the blocked state during the encapsulation process, to prevent other first targets and/or other second targets from entering the droplet, thereby improving the success rate of encapsulation and reducing cell loss caused by co-encapsulation.

In some other embodiments of the present disclosure, the microfluidic system further includes a waste channel and a waste microvalve corresponding to the waste channel. The waste channel is in communication with the pairing channel, and the waste microvalve is configured for controlling opening and closing of the waste channel.

When the pairing microvalve is in the closed state and the first microvalve and the waste microvalve are in the open state, liquid in the first channel flows out through the pairing channel and the waste channel.

Additionally or alternatively, when the pairing microvalve is in the closed state and the second microvalve and the waste microvalve are in the open state, liquid in the second channel flows out through the pairing channel and the waste channel.

In some other embodiments of the present disclosure, the first channel, the second channel, the buffer channel, and the waste channel are all located on a side of the pairing microvalve away from the oil channel.

In some other embodiments of the present disclosure, the first channel and the second channel are located on one side of the pairing channel, and the waste channel is located on another side of the pairing channel.

In some other embodiments of the present disclosure, the microfluidic system further includes at least one of:
at least a first portion of the first channel which is in communication with the pairing channel intersects the pairing channel;
at least a second portion of the second channel which is in communication with the pairing channel intersects the pairing channel; and
at least a third portion of the waste channel which is in communication with the pairing channel intersects the pairing channel.

In some other embodiments of the present disclosure, at least a fourth portion of the buffer channel which is in communication with the pairing channel is arranged coaxially with the pairing channel.

In some other embodiments of the present disclosure, the waste channel is located between the first channel and the second channel in an extending direction of the pairing channel.

In some other embodiments of the present disclosure, the microfluidic system further includes a detection module configured for identifying the single first target in the first channel. After the detection module identifies a single first target, both the first microvalve and the pairing microvalve are in the closed state to keep the single first target in the pairing channel.

Additionally or alternatively, the microfluidic system further includes a detection module configured for identifying the single second target in the second channel. After the detection module identifies the single second target, both the second microvalve and the pairing microvalve are in the closed state to keep the single second target in the pairing channel.

In some other embodiments of the present disclosure, the microfluidic system further includes a waste channel and a waste microvalve corresponding to the waste channel. The waste channel is in communication with the pairing channel, and the waste microvalve is configured for controlling opening and closing of the waste channel.

Before the detection module identifies the single first target, the pairing microvalve is in the closed state and both the first microvalve and the waste microvalve are in the open state, so that liquid in the first channel flows out through the pairing channel and the waste channel.

Additionally or alternatively, before the detection module identifies the single second target, the pairing microvalve is in the closed state and both the second microvalve and the waste microvalve are in the open state, so that liquid in the second channel flows out through the pairing channel and the waste channel.

In some other embodiments of the present disclosure, the detection module includes a camera and a controller configured for controlling the camera to capture an image.

The image includes at least a first image of a first portion of the first channel which is in communication with the pairing channel, and the controller is further configured for identifying the single first target based on the first image.

Additionally or alternatively, the image includes at least a second image of a second portion of the second channel which is in communication with the pairing channel, and the controller is further configured for identifying the single second target based on the second image.

In some other embodiments of the present disclosure, the image includes at least a third image of the pairing channel, and the controller is configured for identifying whether the single first target and/or the single second target is present in the pairing channel based on the third image.

In some other embodiments of the present disclosure, the detection module includes a first light source, a first light detection device, and a controller configured for controlling the first light source to emit first detection light to the first channel. The first detection light is configured for exciting the single first target to emit first fluorescence upon irradiating the single first target. When the first light detection device detects the first fluorescence, the controller identifies presence of the single first target.

Additionally or alternatively, the detection module includes a second light source, a second light detection device, and a controller configured for controlling the second light source to emit second detection light to the second channel. The second detection light is configured for exciting the single second target to emit second fluorescence upon irradiating the single second target. When the second light detection device detects the second fluorescence, the controller identifies presence of the single second target.

In some other embodiments of the present disclosure, the detection module includes a first detection electrode assembly extending into the first channel, and a controller configured for identifying the single first target based on a signal detected by the first detection electrode assembly.

Additionally or alternatively, the detection module includes a second detection electrode assembly extending into the second channel, and a controller configured for identifying the single second target based on a signal detected by the second detection electrode assembly.

In some other embodiments of the present disclosure, after the detection module identifies the single first target, the first microvalve is switched from the open state to the closed state after a time delay during which the single first target enters the pairing channel from the first channel.

Additionally or alternatively, after the detection module identifies the single second target, the second microvalve is switched from the open state to the closed state after a time delay during which the single second target enters the pairing channel from the second channel.

In some other embodiments of the present disclosure, the microfluidic system further includes an injection channel in communication with the oil channel. A communication portion of the pairing channel which is in communication with the oil channel is defined as a first communication portion. A communication portion of the injection channel which is in communication with the oil channel is defined as a second communication portion which is located downstream of the first communication portion in a flow direction of the oil in the oil channel.

The microfluidic system further includes a fusion electrode assembly configured for applying an electric field to the second communication portion to introduce, merge, or fuse liquid in the injection channel into the droplet that passes through the second communication portion.

In some other embodiments of the present disclosure, the microfluidic system includes a processing region in which the first channel, the second channel, the oil channel, the buffer channel, the pairing channel, and the injection channel are all located.

The fusion electrode assembly includes a first electrode surrounding the processing region and a second electrode.

In some other embodiments of the present disclosure, two ends of the first electrode are parallel to the second electrode, and the second electrode is between the two ends of the first electrode.

In some other embodiments of the present disclosure, the microfluidic system further includes an oil microvalve corresponding to the oil channel, and the oil microvalve is configured for controlling opening and closing of the oil channel.

In some other embodiments of the present disclosure, the microfluidic system further includes a first microvalve control channel which includes a membrane between the first channel and the first microvalve control channel, and the membrane is configured to be driven to protrude toward the first channel to enter a closed state.

An inner wall of the first channel is an arc-shaped inner wall.

In some other embodiments of the present disclosure, the microfluidic system includes a microfluidic chip which includes a base layer, a control layer, and a channel layer stacked in sequence.

The first channel, the second channel, the oil channel, the buffer channel, and the pairing channel are arranged in the channel layer.

The pairing microvalve, the first microvalve, the second microvalve, and the buffer microvalve are arranged in the control layer.

In some other embodiments of the present disclosure, the first target is a cell, and the second target is a microsphere.

In some other embodiments of the present disclosure, the microsphere is a magnetic microsphere.

In some other embodiments of the present disclosure, the microsphere is a fluorescent magnetic microsphere.

In some other embodiments of the present disclosure, the first target is a first cell, and the second target is a second cell.

A second embodiment of the present disclosure provides a microfluidic system for target object pairing including a first channel, a second channel, an oil channel, a buffer channel, and a pairing channel. The pairing channel is in communication with the oil channel. The first channel and the second channel are both in communication with the pairing channel. The buffer channel is in communication each of with the first channel and the second channel. The first channel includes a first portion in communication with the pairing channel, and the second channel includes a second portion in communication with the pairing channel.

The microfluidic system further includes a first microvalve corresponding to the first channel, a second microvalve corresponding to the second channel, and a buffer microvalve corresponding to the buffer channel. The first microvalve is configured for controlling opening and closing of the first channel. The second microvalve is configured for controlling opening and closing of the second channel. The buffer microvalve is configured for controlling opening and closing of the buffer channel;

When a single first target is present in the first portion and a single second target is present in the second portion, both the first microvalve and the second microvalve are in a closed state, and the buffer microvalve is in an open state, so that a buffer solution in the buffer channel pushes the single first target in the first portion and the single second target in the second portion into the oil in the oil channel through the pairing channel to form a droplet.

In some other embodiments of the present disclosure, the microfluidic system further includes a waste channel, a droplet channel, a waste microvalve corresponding to the waste channel, and a droplet microvalve corresponding to the droplet channel. The waste channel and the droplet channel are respectively in communication with the oil channel. The waste microvalve is configured for controlling opening and closing of the waste channel, and the droplet microvalve is configured for controlling opening and closing of the droplet channel.

When the first microvalve and the waste microvalve are both in the open state and the droplet microvalve is in the closed state, the liquid in the first channel flows out through the pairing channel, the oil channel, and the waste channel.

Additionally or alternatively, when the second microvalve and the waste microvalve are both in the open state and the droplet microvalve is in the closed state, the liquid in the second channel flows out through the pairing channel, the oil channel, and the waste channel.

In some other embodiments of the present disclosure, the microfluidic system further includes a waste channel, a droplet channel, a waste microvalve corresponding to the waste channel, and a droplet microvalve corresponding to the droplet channel. The waste channel and the droplet channel are respectively in communication with the oil channel. The waste microvalve is configured for controlling opening and closing of the waste channel, and the droplet microvalve is configured for controlling opening and closing of the droplet channel.

When a droplet is formed in the oil in the oil channel, the waste microvalve is in the closed state and the droplet microvalve is in the open state, so that the droplet is discharged through the droplet channel.

In some other embodiments of the present disclosure, the microfluidic system further includes a waste channel, a droplet channel, a waste microvalve corresponding to the waste channel, and a droplet microvalve corresponding to the droplet channel. The waste microvalve is configured for controlling opening and closing of the waste channel, and the droplet microvalve is configured for controlling opening and closing of the droplet channel;

The pairing channel is in communication with an inlet end of the oil channel, and the waste channel and the droplet channel are both in communication with an outlet end of the oil channel.

In some other embodiments of the present disclosure, the microfluidic system further includes a detection module configured for identifying the single first target in the first channel. After the detection module identifies a single first target, the first microvalve is in the closed state to keep the single first target in the first portion.

Additionally or alternatively, the microfluidic system further includes a detection module configured for identifying the single second target in the second channel. After the detection module identifies a single second target, the second microvalve is in the closed state to keep the single second target in the second portion.

A third embodiment of the present disclosure provides a pairing method including following steps.

A single first target from a first liquid carrying first targets is identified, and upon identification of the single first target, the first liquid is stopped so that the single first target stays in a target region.

A single second target from a second liquid carrying second targets is identified, and upon identification of the single second target, the second liquid is stopped so that the single second target stays in the target region.

After the single first target and the single second target both stay in the target region, the single first target and the single second target in the target region are pushed into an oil by a third liquid to form a droplet.

In some other embodiments of the present disclosure, a method of identifying a single first target from a first liquid carrying first targets includes: acquiring an image of a detection region, and identifying the single first target based on image information in the image. The first liquid enters the target region after passing through the detection region.

Additionally or alternatively, a method of identifying a single second target from a second liquid carrying second targets includes: acquiring an image of a detection region, and identifying the single second target based on image information in the image. The second liquid enters the target region after passing through the detection region.

In some other embodiments of the present disclosure, a method of identifying a single first target from a first liquid carrying first targets includes: detecting an optical signal in a detection region, and identifying the single first target based on the detected optical signal. The first liquid enters the target region after passing through the detection region.

Additionally or alternatively, a method of identifying a single second target from a second liquid carrying second targets includes: detecting an optical signal in a detection region, and identifying the single second target based on the detected optical signal. The second liquid enters the target region after passing through the detection region.

In some other embodiments of the present disclosure, a method of identifying a single first target from a first liquid carrying first targets includes: detecting an electrical signal generated by an electrode in a detection region, and identifying the single first target based on the detected electrical signal. The first liquid is allowed to enter the target region after passing through the detection region; and/or

Additionally or alternatively, a method of identifying a single second target from a second liquid carrying second targets includes: detecting an electrical signal generated by an electrode in the detection region, and identifying the single second target based on the detected electrical signal. The second liquid enters the target region after passing through the detection region.

In some other embodiments of the present disclosure, the pairing method further includes: pushing the droplet containing the single first target and the single second target to pass through a fusion region, and applying an electric field to the fusion region to incorporate a fourth liquid into the droplet.

Additional aspects and advantages of the present disclosure will be partly given in and partly apparent from the description below, or understood through practice of the present disclosure.

### BRIEF DESCRIPTION OF DRAWINGS

The present disclosure is further described below with reference to the accompanying drawings and embodiments. In the drawings:
FIG. 1 is a schematic diagram of a pairing system in the related art;
FIG. 2 is a schematic diagram of a microfluidic system according to a first embodiment of the present disclosure;
FIG. 3 is a schematic enlarged view of a pairing channel shown in FIG. 2;
FIG. 4 is a schematic flowchart of pairing a first target with a second target by a microfluidic system according to the first embodiment of the present disclosure;
FIG. 5 is a schematic diagram of a microfluidic system including an electrode-based detection module according to the first embodiment of the present disclosure;
FIG. 6 is a schematic diagram of a microfluidic system including an electrofusion module according to the first embodiment of the present disclosure;
FIG. 7 is a schematic enlarged view of an electrofusion process shown in FIG. 6;
FIG. 8 is a schematic diagram of a microvalve in an open state and a closed state in a microfluidic system according to the first embodiment of the present disclosure;
FIG. 9 is a schematic exploded view of a microfluidic chip according to the first embodiment of the present disclosure; and
FIG. 10 is a schematic diagram of a microfluidic system according to a second embodiment of the present disclosure.

### List of reference numerals:

first channel 1, second channel 2, oil channel 3, waste channel 4, sample channel 5;
microfluidic chip 100, base layer 110, control layer 120, first microvalve 121, membrane 1211, second microvalve 122, oil microvalve 123, buffer microvalve 124, waste microvalve 125, pairing microvalve 126, channel layer 130, first channel 131, first portion 1311, second channel 132, second portion 1321, oil channel 133, first communication portion 1331, second communication portion 1332, buffer channel 134, fourth portion 1341, waste channel 135, third portion 1351, pairing channel 136, injection channel 137, first microvalve control channel 138, first inlet 1391, second inlet 1392, third inlet 1393;
first detection light 210, second detection light 220, first detection electrode assembly 230, second detection electrode assembly 240;
fusion electrode assembly 300, first electrode 310, second electrode 320;
single first target A;
single second target B;
droplet C.

### DETAILED DESCRIPTION

Embodiments of the present disclosure will be described in detail hereinafter in conjunction with accompanying drawings in which the same or like reference numerals refer to the same or like elements or elements having the same or like functions throughout. The embodiments described below by reference to the accompanying drawings are illustrative and are intended for illustrating only and are not to be construed as limiting the present disclosure.

In the description of the present disclosure, it should be understood that for the description of orientations, the orientation or positional relationships indicated by the terms such as "up," "down," "front," "rear," "left," "right" and the like are based on orientation or positional relationships shown in the accompanying drawings, and are used only for ease and brevity of illustration and description, rather than indicating or implying that the mentioned apparatus or element must have a particular orientation or must be constructed and operated in a particular orientation. Therefore, such terms should not be construed as limiting of the present disclosure.

In the description of the present disclosure, the term "at least one" means one or more, the term "plurality of/multiple" means at least two, the term such as "greater than," "less than," "exceed" or variants thereof prior to a number or series of numbers is understood to not include the number adjacent to the term. The term such as "above," "below," "within," etc., are understood to include the number adjacent to the term. If used herein, the terms such as "first," "second," and the like are merely used for distinguishing technical features, and are not intended to indicate or imply relative importance, or implicitly point out the number of the indicated technical features, or implicitly point out the precedence order of the indicated technical features.

In the description of the present disclosure, unless otherwise explicitly defined, the terms such as "configure," "install/mount" and "connect" should be understood in a broad sense, and those having ordinary skills in the art can reasonably determine the specific meanings of the above terms in the present disclosure based on the specific contents of the technical scheme.

In the description of the specification, the description with reference to the terms "an embodiment," "some embodiments," "exemplary embodiment," "example," "specific example", or "some examples" and so on means that specific features, structures, materials or characteristics described in connection with the embodiment or example are embraced in at least one embodiment or example of the present disclosure. In this specification, exemplary descriptions of the foregoing terms do not necessarily refer to the same embodiment or example. Moreover, the described specific features, structures, materials, or characteristics may be combined in any suitable manner in one or more embodiments.

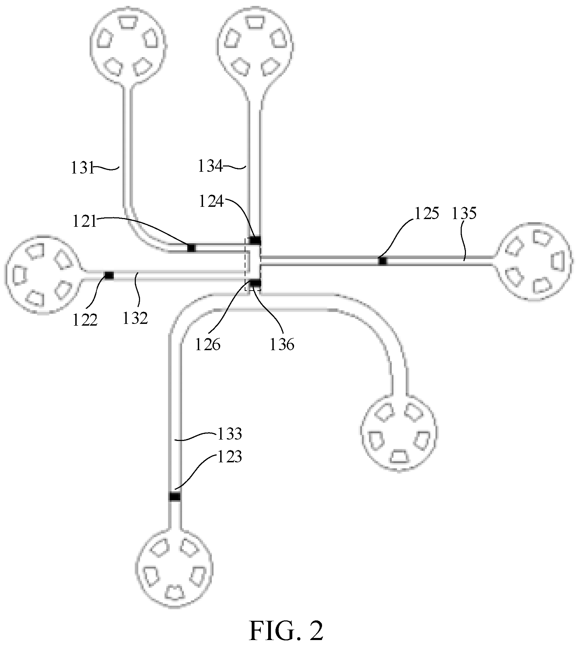

A first embodiment of the present disclosure provides a microfluidic system for target object pairing. Referring to FIG. 2 to FIG. 4, the microfluidic system includes a first channel 131, a second channel 132, an oil channel 133, a buffer channel 134, and a pairing channel 136. The first channel 131 is configured for allowing a first liquid containing first targets to pass through. The second channel 132 is configured for allowing a second liquid containing second targets to pass through. The buffer channel 134 is configured for allowing a buffer solution to pass through. The oil channel 133 is configured for allowing an oil which is immiscible with the first liquid, the second liquid, and the buffer solution to pass through. The pairing channel 136 is configured for allowing a single first target and a single second target to stay therein. The pairing channel 136 is in communication with the oil channel 133. The first channel 131, the second channel 132, and the buffer channel 134 are all in communication with the pairing channel 136. In some embodiments, the microfluidic system further includes a waste channel 135 configured for discharging the first liquid and the second liquid. In this embodiment, an example where the first target is a cell and the second target is a microsphere is described. It can be understood by those having ordinary skill in the art that the first target may be a cell, and the second target may also be a cell.

The microfluidic system of this embodiment further includes a plurality of microvalves, specifically, a pairing microvalve 126 corresponding to the pairing channel 136, a first microvalve 121 corresponding to the first channel 131, a second microvalve 122 corresponding to the second channel 132, and a buffer microvalve 124 corresponding to the buffer channel 134. The pairing microvalve 126 is configured for controlling opening and closing of the pairing channel 136. The first microvalve 121 is configured for controlling opening and closing of the first channel 131. The second microvalve 122 is configured for controlling opening and closing of the second channel 132. The buffer microvalve 124 is configured for controlling opening and closing of the buffer channel 134. Taking the first channel 131 and the first microvalve 121 as an example, the first microvalve 121 controlling opening and closing of the first channel 131 means that the first microvalve 121 has an open state and a closed state. When the first microvalve 121 is in the open state, the first channel 131 is in an unblocked state allowing the first liquid to flow in the first channel 131. When the first microvalve 121 is in the closed state, the first channel 131 is in a blocked state, preventing the first liquid from flowing in the first channel 131.

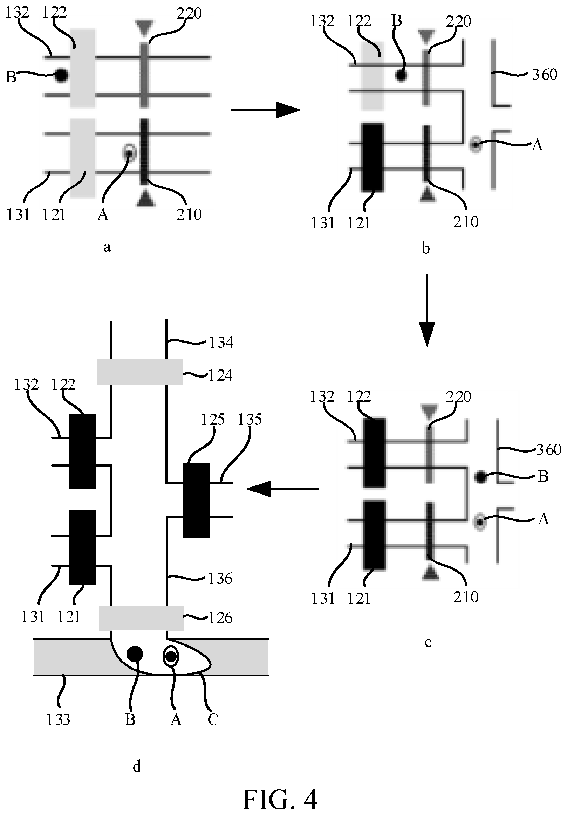

In this embodiment, referring to FIG. 4, when a single first target A and a single second target B are present in the pairing channel 136, both the first microvalve 121 and the second microvalve 122 are in a closed state (a microvalve filled with black indicates that it is in the closed state), and both the pairing microvalve 126 and the buffer microvalve 124 are in an open state (a microvalve filled with gray indicates that it is in the open state). In this case, the buffer solution in the buffer channel 134 can push the single first target A and the single second target B in the pairing channel 136 into the oil in the oil channel 133 to form a droplet C.

As can be seen from the above, in this embodiment, a separate pairing channel 136 is configured to temporarily store a single first target A and a single second target B, and a separate buffer channel 134 is configured to utilize the buffer solution to propel the single first target A and the single second target B into the oil. This obviates the need to propel the single first target A and the single second target B through the first liquid and the second liquid, so that the first channel 131 and the second channel 132 can be kept in the blocked state during the encapsulation process, to prevent other first targets and/or other second targets from entering the droplet C, thereby improving the success rate of encapsulation and reducing cell loss caused by co-encapsulation.

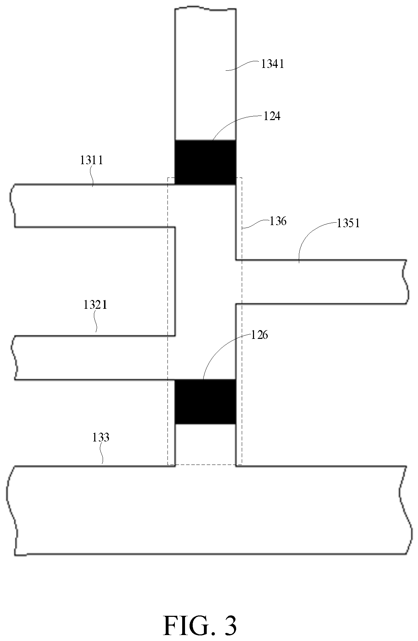

Based on the first embodiment, referring to FIG. 2 and FIG. 3, in some embodiments of the present disclosure, the microfluidic system further includes a waste channel 135 configured for discharging the first liquid and the second liquid, and the waste channel 135 is in communication with the pairing channel 136. In addition, the microfluidic system further includes a waste microvalve 125 corresponding to the waste channel 135, and the waste microvalve 125 is configured for controlling opening and closing of the waste channel 135. When the pairing microvalve 126 is in the closed state and the first microvalve 121 and the waste microvalve 125 are in the open state, the first liquid in the first channel 131 is allowed to flow out through the pairing channel 136 and the waste channel 135. It should be noted that the first target gradually approaches and reaches the pairing channel 136 following the flow of the first liquid. Before the first target reaches the pairing channel 136, the first channel 131 continuously discharges the first liquid, which needs to be discharged as waste liquid. In this embodiment, the first liquid discharged from the first channel 131 first enters the pairing channel 136, and then enters the waste channel 135 to be discharged. In other words, in this embodiment, the discharged first liquid does not flow through the oil channel 133. As compared with the scheme shown in FIG. 1, the scheme of this embodiment has the following advantages.
1. In the scheme shown in FIG. 1, the discharged waste liquid has to flow through the oil channel 3, and therefore will inevitably take away the oil in the oil channel 3, resulting in a waste of oil. In contrast, in this embodiment, the discharged first liquid and second liquid do not flow through the oil channel 133, and therefore will not take away the oil in the oil channel 133, so that the amount of oil needing to be used can be reduced.
2. In the scheme shown in FIG. 1, the discharged waste liquid has to flow through the oil channel 3, so the oil channel 3 has to be blocked during pairing. After the pairing is completed, the first channel 1, the second channel 2, and the oil channel 3 have to be unblocked synchronously, otherwise the oil channel 3 will not provide the oil in time and droplets cannot be formed. Therefore, the scheme shown in FIG. 1 has very high requirements on the control of the microvalves corresponding to the first channel 1, the second channel 2, and the oil channel 3, respectively, leading to a risk of failure of target encapsulation as well as the loss of targets. In contrast, in this embodiment, because the discharged first liquid and second liquid do not flow through the oil channel 133, the oil channel 133 is always filled up with the oil. After the pairing is completed, only the buffer microvalve 124 and the pairing microvalve 126 have to be opened. Even if the buffer microvalve 124 and the pairing microvalve 126 are not opened synchronously, the encapsulation will not fail.
3. In the scheme shown in FIG. 1, after the oil channel 3 is blocked, some oil will still remain inside, and an empty droplet will be formed when the waste liquid flows through the residual oil. Because the empty droplet will contaminate normal droplets, the empty droplet and the normal droplet have to be distributed to different channels through the joint action of the microvalve of the waste channel 4 and the microvalve of the sample channel 5, which also poses very high requirements on the control of the two microvalves; otherwise, a loss will be caused. For example, if the microvalve of the waste channel 4 is not closed in time or the microvalve of the sample channel 5 is not opened in time after a droplet is formed, the successfully encapsulated droplet will also enter the waste channel 4. In contrast, in this embodiment, because the discharged first liquid and second liquid do not flow through the oil channel 133, an encapsulated droplet C can reach a subsequent storage position following the flow of the oil in the oil channel 133, which obviates the need of subsequent channel selection, thereby reducing the control difficulty and avoiding the loss of targets caused by the unduly high control difficulty.
4. As described above, in the scheme shown in FIG. 1, a large number of empty droplets are formed, and these droplets have to be sorted by switching of the valves, thereby greatly affecting the processing speed. As compared with the scheme illustrated in FIG. 1, the microfluidic chip structure of this embodiment avoids the formation of a large number of empty droplets, and obviates the need to sort empty droplets from sample droplets, thereby improving the pairing efficiency. For example, 5 to 10 droplets can be formed per second, i.e., 18,000 to 36,000 droplets can be formed per hour. In addition, the microfluidic chip structure of this embodiment can also significantly reduce the control difficulty of the microvalves.

In some embodiments, the microfluidic system further includes a power element, such as a pump, configured for driving the buffer solution to flow. In some embodiments, the microfluidic system includes a high-speed pump configured for driving the buffer solution to flow at a high speed and stopping the buffer solution, to adapt to high-speed pairing.

Based on the first embodiment, referring to FIG. 2 and FIG. 3, in some embodiments of the present disclosure, the waste channel 135 is in direct communication with the pairing channel 136, and the microfluidic system further includes a waste microvalve 125 corresponding to the waste channel 135. The waste microvalve 125 is configured for controlling opening and closing of the waste channel 135. When the pairing microvalve 126 is in the closed state and both the second microvalve 122 and the waste microvalve 125 are in the open state, the second liquid in the second channel 132 is allowed to flow out through the pairing channel 136 and the waste channel 135. The discharge of the second liquid, can refer to the discharge of the first liquid, and the details will not be repeated herein.

When the waste channel 135 is in direct communication with the pairing channel 136, referring to FIG. 2 and FIG. 3, in some embodiments of the present disclosure, the first channel 131, the second channel 132, the buffer channel 134, and the waste channel 135 are all located on a side of the pairing microvalve 126 away from the oil channel 133, to prevent the first liquid, the second liquid, etc. from entering the oil channel 133 before the pairing is completed. In the embodiment as shown in the figures, the oil channel 133 is distributed in a horizontal direction, and the pairing channel 136 is distributed in a vertical direction with a lower end thereof being in communication with the oil channel 133. In this case, the pairing microvalve 126 is substantially located at the lower end of the pairing channel 136, and the first channel 131, the second channel 132, the buffer channel 134, and the waste channel 135 are all located on an upper side of the pairing microvalve 126.

The control of the microvalves is described below with reference to the above configuration.

### 1. Pairing process

The buffer microvalve 124 and the pairing microvalve 126 are closed, and the first microvalve 121, the second microvalve 122, and the waste microvalve 125 are opened, so that the first liquid and the second liquid are discharged through the pairing channel 136 and the waste channel 135. After a single first target is identified, the first microvalve 121 is closed, so that the single first target stays in the pairing channel 136 (e.g., as illustrated in a sub-figure b in FIG. 4). After a single second target is identified, the second microvalve 122 is closed, so that the single second target stays in the pairing channel 136 (e.g., as illustrated in a sub-figure c in FIG. 4). After both the single first target and the single second target stay in the pairing channel 136, the waste microvalve 125 is closed (e.g., as illustrated in a sub-figure c in FIG. 4).

### 2. Encapsulation process

After the first microvalve 121, the second microvalve 122, and the waste microvalve 125 are closed, and the buffer microvalve 124 and the pairing microvalve 126 are opened, so that the buffer solution is able to push the single first target and the single second target in the pairing channel 136 into the oil in the oil channel 133 to form a droplet (e.g., as illustrated in a sub-figure d in FIG. 4).

When the waste channel 135 is in direct communication with the pairing channel 136, referring to FIG. 3, in some embodiments of the present disclosure, the waste channel 135 is located between the first channel 131 and the second channel 132 in an extending direction of the pairing channel 136, so that the discharge of the first liquid does not cause the still single second target B to move, and the discharge of the second liquid does not cause the still single first target A to move. Therefore, the discharge of the first liquid and the discharge of the second liquid may be performed synchronously.

When the waste channel 135 is in direct communication with the pairing channel 136, referring to FIG. 3, in some embodiments of the present disclosure, the first channel 131 and the second channel 132 are located on one side of the pairing channel 136, and the waste channel 135 is located on an other side of the pairing channel 136. In the embodiment shown in FIG. 3, both the first channel 131 and the second channel 132 are located on a left side of the pairing channel 136, and the waste channel 135 is located on a right side of the pairing channel 136, so that the first liquid and the second liquid can flow in substantially identical direction, thereby facilitating the discharge of the waste liquid.

When the waste channel 135 is in direct communication with the pairing channel 136, referring to FIG. 3, in an embodiment of the present disclosure, at least a first portion 1311 of the first channel 131 which is in communication with the pairing channel 136 is configured to intersect the pairing channel 136. In the embodiment shown in FIG. 3, the first portion 1311 is perpendicular to the pairing channel 136.

When the waste channel 135 is in direct communication with the pairing channel 136, referring to FIG. 3, in an embodiment of the present disclosure, at least a second portion 1321 of the second channel 132 which is in communication with the pairing channel 136 is configured to intersect the pairing channel 136. In the embodiment shown in FIG. 3, the second portion 1321 is perpendicular to the pairing channel 136.

When the waste channel 135 is in direct communication with the pairing channel 136, referring to FIG. 3, at least a third portion 1351 of the waste channel 135 which is in communication with the pairing channel 136 is configured to intersect the pairing channel 136. In the embodiment shown in FIG. 3, the third portion 1351 is perpendicular to the pairing channel 136.

When the waste channel 135 is in direct communication with the pairing channel 136, referring to FIG. 3, at least a fourth portion 1341 of the buffer channel 134 which is in communication with the pairing channel 136 is arranged coaxially with the pairing channel 136, so that the buffer solution can enter the pairing channel 136 in the same direction and push a single first target A and a single second target B into the oil. It should be noted that in this embodiment, the buffer channel 134 and the pairing channel 136 may be two parts of a whole channel. Taking FIG. 3 as an example, a lower part of a vertical channel is the pairing channel 136, and an upper part of the vertical channel is the buffer channel 134. For ease of understanding, the range of the pairing channel 136 is roughly marked by a dashed line box in the figure, but the dashed line box should not be construed as a specific limitation on the shape and length of the pairing channel 136.

It can be understood that the above embodiments may be combined, e.g., the first portion 1311, the second portion 1321, and the third portion 1351 are all disposed intersecting the pairing channel 136, and the fourth portion 1341 is arranged coaxially with the pairing channel 136.

Based on the first embodiment, in some embodiments of the present disclosure, the microfluidic system further includes a detection module configured for identifying a single first target A in the first channel 131. After the detection module identifies a single first target A, both the first microvalve 121 and the pairing microvalve 126 are in the closed state to keep the single first target A in the pairing channel 136. It should be noted that in this embodiment, the first microvalve 121 being in the closed state means switching from the open state to the closed state, and the pairing microvalve 126 being in the closed state means remaining in the closed state.

In some other embodiments, the detection module is configured for identifying a single second target B in the second channel 132. After the detection module identifies a single second target B, both the second microvalve 122 and the pairing microvalve 126 are in the closed state to keep the single second target B in the pairing channel 136. It should be noted that in this embodiment, the second microvalve 122 being in the closed state means switching from the open state to the closed state, and the pairing microvalve 126 being in the closed state means remaining in the closed state.

In some embodiments of the present disclosure, the microfluidic system further includes a waste microvalve 125 corresponding to the waste channel 135. The waste microvalve 125 is configured for controlling opening and closing of the waste channel 135 when the detection module identifies a single first target A. Before the detection module identifies a single first target A, the pairing microvalve 126 is in the closed state and the first microvalve 121 and the waste microvalve 125 are in the open state, allowing the first liquid in the first channel 131 to flow out through the pairing channel 136 and the waste channel 135. It should be noted that in this embodiment, the pairing microvalve 126 being in the closed state means remaining in the closed state, and the first microvalve 121 and the waste microvalve 125 being in the open state means remaining in the open state.

When the detection module is detecting a single second target B, in some embodiments of the present disclosure, before the detection module identifies a single second target B, the pairing microvalve 126 is in the closed state and the second microvalve 122 and the waste microvalve 125 are in the open state, allowing the second liquid in the second channel 132 to flow out through the pairing channel 136 and the waste channel 135. It should be noted that in this embodiment, the pairing microvalve 126 being in the closed state means remaining in the closed state, and the second microvalve 122 and the waste microvalve 125 being in the open state means remaining in the open state.

In some embodiments of the present disclosure, the detection module is a visual detection module including a camera and a controller, and the controller is configured for controlling the camera to capture an image when the detection module is detecting a single first target A. In this embodiment, the image captured by the camera includes at least a first image of the first portion 1311 of the first channel 131 which is in communication with the pairing channel 136, and the controller is further configured for identifying the single first target A based on the first image after the camera captures the image. In other words, an operation of identifying the single first target A has been completed before the single first target A enters the pairing channel 136. It should be noted that the microsphere in this embodiment may be a magnetic microsphere that can be adsorbed by a magnetic member for recovery.

In some other embodiments, the image captured by the camera includes at least a second image of the second portion 1321 of the second channel 132 which is in communication with the pairing channel 136, and the controller is further configured for identifying the single second target B based on the second image. In other words, an operation of identifying the single second target B has been completed before the single second target B enters the pairing channel 136.

In the above process, after the image captured by the camera is transmitted to the controller, the controller performs object detection in a detection region (e.g., a region having a length of 200 pixels and a width of 200 pixels), classifies cropped object images using a model trained by a convolutional neural network, makes a detection according to a result of the classification, and controls the corresponding microvalves.

In some embodiments of the present disclosure, the image captured by the camera further includes at least a third image of the pairing channel 136, and the controller is configured for identifying whether the single first target A is present in the pairing channel 136 based on the third image when the detection module is detecting a single first target A based on an image. In other words, in this embodiment, the image captured by the camera can also be used to verify whether a single first target A has entered the pairing channel 136.

In some other embodiments, the image captured by the camera further includes at least a third image of the pairing channel 136, and the controller is configured for identifying whether the single second target B is present in the pairing channel 136 based on the third image. In other words, in this embodiment, the image captured by the camera can also be used to verify whether a single second target B has entered the pairing channel 136.

It should be noted that the above embodiments may be combined, e.g., the image captured by the camera includes the first image, the second image, and the third image, in order to enable the controller to execute a combination of operations based on the same image. For example, the controller can identify a single first target A and a single second target B at the same time. Alternatively, it can identify a single first target A and determine whether a single second target B has reached the pairing channel 136. Alternatively, it can identify a single second target B and determine whether a single first target A has reached the pairing channel 136. Alternatively, it can determine whether a single first target A and a single second target B have reached the pairing channel 136.

In some embodiments of the present disclosure, the detection module is a fluorescence detection module, which includes a first light source, a first light detection device, and a controller. The controller is configured for controlling the first light source to emit first detection light 210 to the first channel 131, when the detection module is detecting a single first target A. The first detection light 210 is configured for exciting the single first target A to emit first fluorescence upon irradiating the single first target A. The first light detection device is configured for receiving the first fluorescence excited by the single first target A. For example, the first light source is a laser emitter, and the first light detection device is a photomultiplier tube. After receiving the first fluorescence emitted by the single first target A, the photomultiplier tube can convert the first fluorescence from an optical signal into an electrical signal which is in turn transmitted to the controller. It should be noted that the microsphere in this embodiment may be a fluorescent magnetic microsphere dyed with a fluorescent substance, so that it can emit fluorescence when excited by an external light source.

Referring to FIG. 4, the first light source projects the first detection light 210 to a specified position in the first channel 131. When no cell reaches the specified position, no optical signal will be received by the first light detection device. When a cell reaches the specified position, the cell is excited by the first detection light 210 to emit fluorescence. When the first light detection device detects the first fluorescence, the controller identifies the presence of the single first target A.

In some embodiments of the present disclosure, the detection module is specifically a fluorescence detection module, which includes a second light source, a second light detection device, and a controller. The controller is configured for controlling the second light source to emit second detection light 220 to the second channel 132 when the detection module is detecting a single second target B. The second detection light 220 is configured for exciting the single second target B to emit second fluorescence upon irradiating the single second target B. When the second light detection device detects the second fluorescence, the controller identifies the presence of the single second target B.

A fluorescence-based scheme for identifying the first target A and/or the second target B is described below with reference to FIG. 4.

### 1. Identification of first target A

The first microvalve 121 is in the open state, allowing the first liquid to continuously flow through a first detection position irradiated by the first detection light 210. When the single first target A passes through the first detection position along with the first liquid, the single first target A is excited by the first detection light 210 to emit first fluorescence. Upon receiving the first fluorescence, the first light detection device generates an electrical signal. The controller identifies the presence of the single first target A based on the electrical signal, and then controls the first microvalve 121 to switch to the closed state, so that the single first target A stays in the pairing channel 136.

### 2. Identification of second target B

The second microvalve 122 is in the open state, allowing the second liquid to continuously flow through a second detection position irradiated by the second detection light 220. When the single second target B passes through the second detection position along with the second liquid, the single second target B is excited by the second detection light 220 to emit second fluorescence. Upon receiving the second fluorescence, the second light detection device generates an electrical signal. The controller identifies the presence of the single second target B based on the electrical signal, and then controls the second microvalve 122 to switch to the closed state, so that the single second target B stays in the pairing channel 136.

It should be noted that the first light source and the second light source may be laser emitters having different wavelengths.

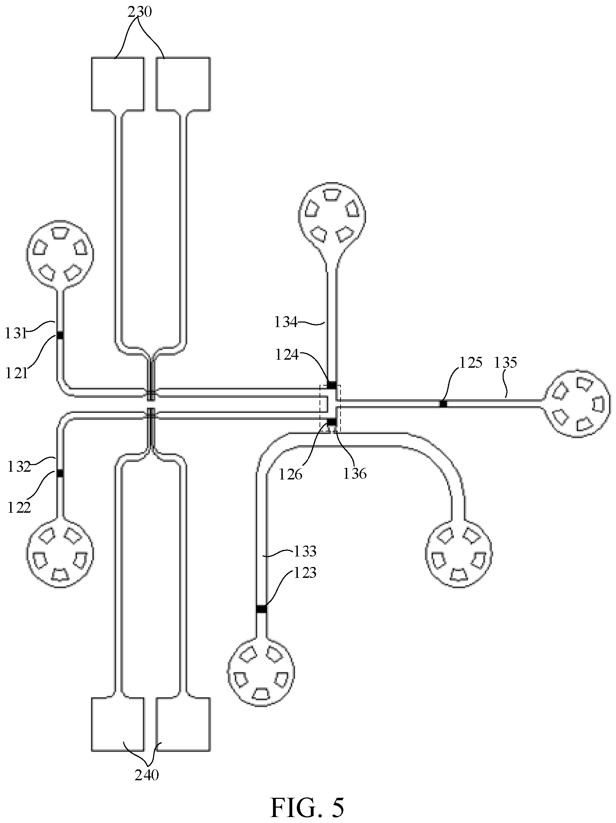

In some embodiments of the present disclosure, the detection module is an electrode-based detection module. Referring to FIG. 5, the detection module includes a first detection electrode assembly 230 and a controller. The first detection electrode assembly 230 extends into the first channel 131 when the detection module is detecting a single first target A. When a single first target A passes by the first detection electrode assembly 230, the first detection electrode assembly 230 generates a corresponding signal, and the controller is configured for identifying the single first target A based on the signal detected by the first detection electrode assembly 230. For example, the first detection electrode assembly 230 includes a positive electrode and a negative electrode which are arranged in parallel. When the microfluidic system includes a microfluidic chip 100, the first detection electrode assembly 230 may be a metal layer between a control layer 120 and a channel layer 130 of the microfluidic chip 100. In addition, in this embodiment, the signal detected by the first detection electrode assembly 230 may be a change in amplitude and phase difference. It should be noted that the microsphere in this embodiment may be a magnetic microsphere that can be adsorbed by a magnetic member for recovery.

In some embodiments of the present disclosure, the detection module is an electrode-based detection module. Referring to FIG. 5, the detection module includes a second detection electrode assembly 240 and a controller. The second detection electrode assembly 240 extends into the second channel 132 when the detection module is detecting a single second target B. The controller is configured for identifying the single second target B based on the signal detected by the second detection electrode assembly 240. For details of the second detection electrode assembly 240, reference can be made to the description of the first detection electrode assembly 230.

In some embodiments of the present disclosure, after the detection module identifies the single first target A, the first microvalve 121 is switched from the open state to the closed state after a specified time delay, so that there is sufficient time for the single first target A to enter the pairing channel 136 from the first channel 131 when the detection module is detecting a single first target A. Referring to FIG. 4 and FIG. 5, the identification position at which the single first target A is identified is at a certain distance from the pairing channel 136, and delaying the closing of the first microvalve 121 in this embodiment ensures that the single first target A can enter the pairing channel 136. It should be noted that the specified time delay may be determined according to the distance between the identification position and the pairing channel 136 and the flow rate of the first liquid.

In some embodiments of the present disclosure, after the detection module identifies the single second target B, the second microvalve 122 is switched from the open state to the closed state after a specified time delay, so that there is sufficient time for the single second target B to enter the pairing channel 136 from the second channel 132 when the detection module is used to identify a single second target B. Referring to FIG. 4 and FIG. 5, the identification position at which the single second target B is identified is at a certain distance from the pairing channel 136, and delaying the closing of the second microvalve 122 in this embodiment ensures that the single second target B can enter the pairing channel 136. It should be noted that the specified time delay may be determined according to the distance between the identification position and the pairing channel 136 and the flow rate of the second liquid.

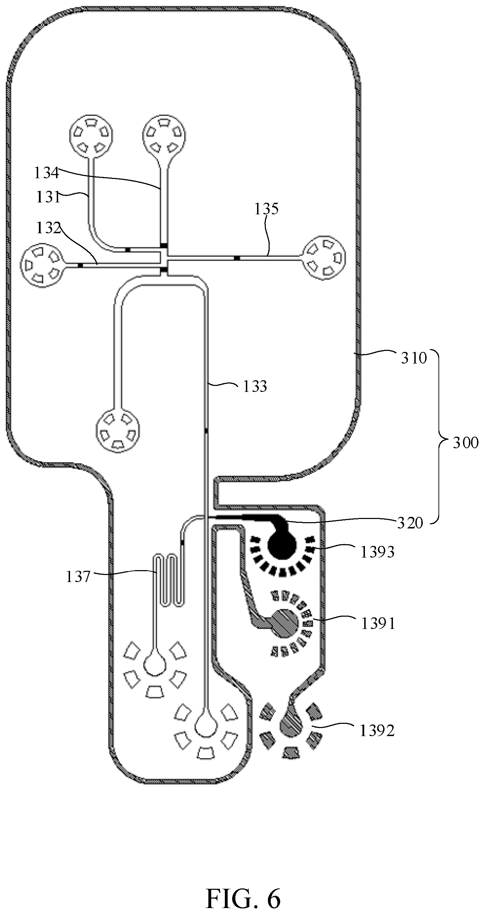

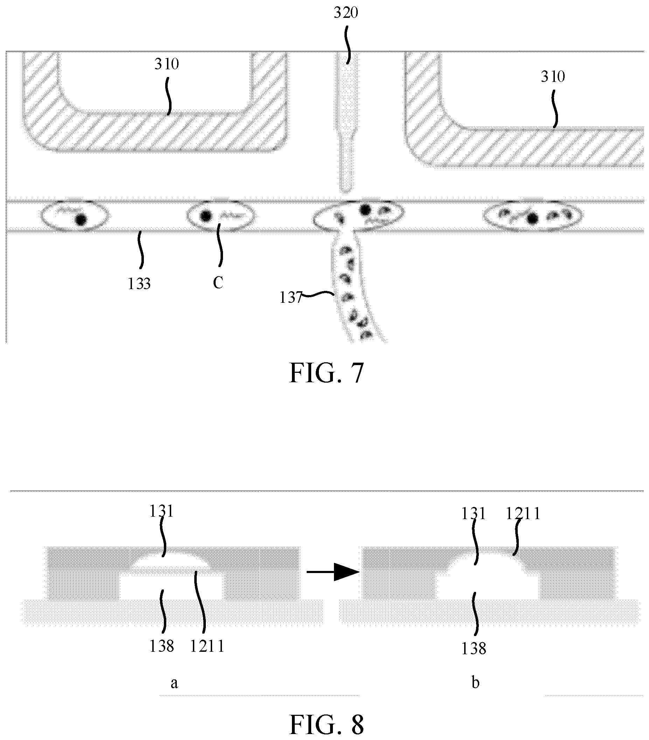

Based on the first embodiment, referring to FIG. 6 and FIG. 7, in some embodiments of the present disclosure, the microfluidic system further includes an injection system configured for injecting liquid into the droplet C, e.g., injecting a reverse transcription mix into the droplet C. For example, the microfluidic system further includes an injection channel 137 which is in communication with the oil channel 133. A communication portion of the pairing channel 136 which is in communication with the oil channel 133 is defined as a first communication portion 1331. A communication portion of the injection channel 137 which is in communication with the oil channel 133 is defined as a second communication portion 1332 which is located downstream of the first communication portion 1331 in a flow direction of the oil in the oil channel 133.

The microfluidic system further includes a fusion electrode assembly 300 configured for applying an electric field to the second communication portion 1332. When the droplet C passes through the second communication portion 1332, the liquid in the injection channel 137 is incorporated into the droplet C under action of the electric field. The liquid in the injection channel 137 is also immiscible with the oil, and pressure in the injection channel 137 is small, so that the liquid in the injection channel 137 slightly protrudes into the oil channel 133. As such, when passing through the second communication portion 1332, the droplet C can come into contact with the liquid in the injection channel 137, thereby facilitating the fusion of the droplet C and the liquid in the injection channel 137.

When the microfluidic system further includes the injection system, referring to FIG. 6 and FIG. 7, in some embodiments of the present disclosure, the channel layer 130 includes a processing region, and the first channel 131, the second channel 132, the oil channel 133, the buffer channel 134, the waste channel 135, the pairing channel 136, and the injection channel 137 are all located in the processing region. It should be noted that the processing region is a virtual region defined to facilitate understanding, and does not mean that the microfluidic system must have a region that is clearly bounded and can be identified.

In this embodiment, the fusion electrode assembly 300 includes a first electrode 310 and a second electrode 320. The first electrode 310 surrounds the processing region to achieve a good electrofusion effect. In some embodiments, when the microfluidic system includes a microfluidic chip 100, a channel layer 130 of the microfluidic chip 100 has an electrode channel into which a metal rod is inserted. The metal rods are heated to melt and fill the electrode channel, and the molten metal rods are cooled to form the first electrode 310 and the second electrode 320 respectively. In some embodiments, the electrode channel has an electrode inlet for inserting the respective metal rod therein. Taking FIG. 6 as an example, the electrode inlet includes a first inlet 1391 and a second inlet 1392 at two ends of a first electrode channel, and a third inlet 1393 at one end of a second electrode channel. It should be noted that the first electrode channel in this embodiment is long, and when the metal rod is inserted through one of the inlets, the other injection port can serve as an exhaust port, allowing the molten metal to fill up the entire electrode channel.

When the fusion electrode assembly 300 includes the first electrode 310 and the second electrode 320 and the first electrode 310 surrounds the processing region, referring to FIG. 7, in some embodiments of the present disclosure, two ends of the first electrode 310 are parallel to the second electrode 320, and the second electrode 320 is arranged between the two ends of the first electrode 310 to achieve a good electrofusion effect.

Based on the first embodiment, referring to FIG. 2, in some embodiments of the present disclosure, the microfluidic chip 100 further includes an oil microvalve 123 corresponding to the oil channel 133. The oil microvalve 123 is configured for controlling opening and closing of the oil channel 133. When the oil microvalve 123 is opened, the oil in the oil channel 133 flows to drive the droplet C to move downstream. In the embodiment shown in the figures, the oil microvalve 123 is located upstream of the first communication portion 1331 in a flow direction of the oil in the oil channel 133.

Based on the first embodiment, in some embodiments of the present disclosure, the microfluidic system further includes a first microvalve control channel 138. Referring to FIG. 8, the first microvalve 121 includes a membrane 1211 between the first channel 131 and the first microvalve control channel 138. When the first microvalve 121 is in the open state as shown in a sub-figure a in FIG. 8, the membrane 1211 is in a horizontal state, allowing the first liquid to flow in the first channel 131. When the membrane 1211 is driven to protrude toward the first channel 131 to enter a closed state as shown in a sub-figure b in FIG. 8, the membrane 1211 comes into contact with an inner wall of the first channel 131 to block the first channel 131, thereby preventing the first liquid from flowing in the first channel 131.

In this embodiment, the inner wall of the first channel 131 is an arc-shaped inner wall, so that the membrane 1211 protrudes toward the first channel 131 to fit the inner wall, thereby ensuring the blocking effect.

It should be noted that in addition to the first microvalve control channel 138, the microfluidic system further includes one or more microvalve control channels respectively corresponding to the second microvalve 122, the oil microvalve 123, the buffer microvalve 124, the waste microvalve 125, and the pairing microvalve 126. Correspondingly, the second microvalve 122, the oil microvalve 123, the buffer microvalve 124, the waste microvalve 125, and the pairing microvalve 126 each include a membrane. For the specific configuration and control mode, reference can be made to the description of the first microvalve control channel 138, and the details will not be repeated herein.

It should also be noted that power that drives the membrane 1211 to protrude may be gas pressure. In this embodiment, the first microvalve control channel 138 is filled with liquid, and an external gas source drives the liquid in the first microvalve control channel 138 to flow, thus pushing the membrane 1211 to protrude or reset. As compared with a method of directly driving the membrane 1211 by gas pressure, because the liquid is difficult to compress, this method can achieve the rapid response of the membrane 1211, thereby adapting to high-speed pairing.

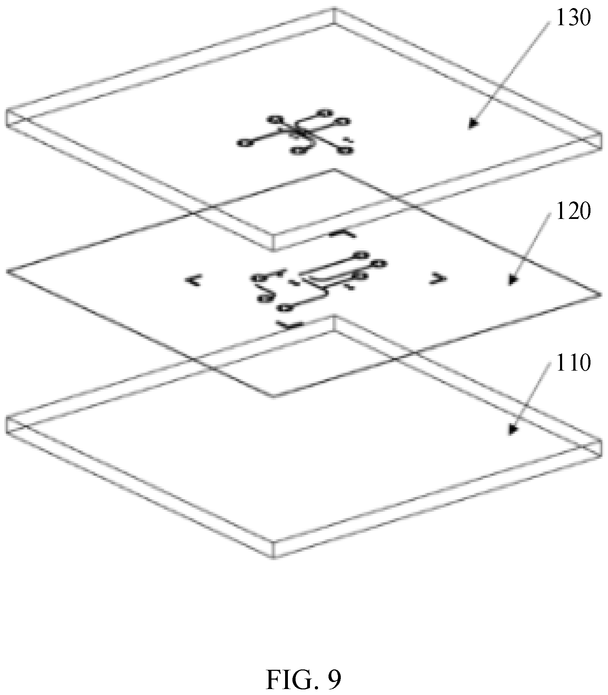

Based on the first embodiment, referring to FIG. 9, in some embodiments of the present disclosure, the microfluidic system includes a microfluidic chip 100. The microfluidic chip 100 includes a base layer 110, a control layer 120, and a channel layer 130 stacked in sequence. The base layer 110 may be made of glass. The control layer 120 and the channel layer 130 may be made of polydimethylsiloxane (PDMS).

The channel layer 130 has the first channel 131, the second channel 132, the oil channel 133, the buffer channel 134, the waste channel 135, and the pairing channel 136 therein. The control layer 120 has the pairing microvalve 126, the first microvalve 121, the second microvalve 122, and the buffer microvalve 124, and the respective microvalve control channels corresponding to the microvalves.

Based on the first embodiment, in some embodiments of the present disclosure, the first target A is a cell, and the second target B is a microsphere. In some embodiments, the microsphere may be further configured to be a magnetic microsphere, so that RNA released after cell lysis can be adsorbed on the surface of the microsphere, and subsequently the microsphere may be collected by a magnetic member to in turn collect the RNA. In some embodiments, the microsphere may be further configured to be a fluorescent magnetic microsphere which can emit fluorescence when excited by external light.

Based on the first embodiment, in some embodiments of the present disclosure, the first target A is a first cell, and the second target B is a second cell, so that it is intended to study the interaction effect between cells.

A second embodiment of the present disclosure provides a microfluidic system for target object pairing. A difference between the second embodiment and the first embodiment lies in that in the microfluidic system of the first embodiment, the first target A and the second target B stay in the pairing channel 136; while in this embodiment, the first target A and the second target B respectively stay in the first channel 131 and the second channel 132.

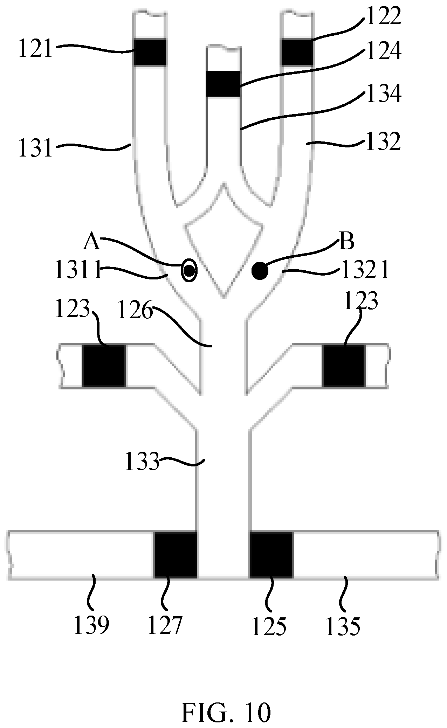

Referring to FIG. 10, the microfluidic system includes a first channel 131, a second channel 132, an oil channel 133, a buffer channel 134, and a pairing channel 136. The first channel 131 is configured for allowing a first liquid containing first targets to pass through. The second channel 132 is configured for allowing a second liquid containing second targets to pass through. The buffer channel 134 is configured for allowing a buffer solution to pass through. The oil channel 133 is configured for allowing an oil which is immiscible with the first liquid, the second liquid, and the buffer solution to pass through. The pairing channel 136 is in communication with the oil channel 133. The first channel 131 and the second channel 132 are both in communication with the pairing channel 136. The buffer channel 134 is respectively in communication with the first channel 131 and the second channel 132. The first channel 131 includes a first portion 1311 in communication with the pairing channel. The second channel 132 includes a second portion 1321 in communication with the pairing channel. In some further embodiments, the microfluidic system further includes a waste channel 135 configured for discharging the first liquid and the second liquid.

The microfluidic system of this embodiment further includes a plurality of microvalves, such as a first microvalve 121 corresponding to the first channel 131, a second microvalve 122 corresponding to the second channel 132, and a buffer microvalve 124 corresponding to the buffer channel 134. The first microvalve 121 is configured for controlling opening and closing of the first channel 131. The second microvalve 122 is configured for controlling opening and closing of the second channel 132. The buffer microvalve 124 is configured for controlling opening and closing of the buffer channel 134. The meanings of opening and closing are identical to those described in the first embodiment.

In this embodiment, referring to FIG. 10, when a single first target A is present in the first portion 1311 and a single second target B is present in the second portion 1321, the first microvalve 121 and the second microvalve 122 are in the closed state and the buffer microvalve 124 is in the open state, so that the buffer solution in the buffer channel 134 respectively pushes the single first target A in the first portion 1311 and the single second target B in the second portion 1321 into the pairing channel 136, and then pushes the single first target A and the single second target B together into the oil in the oil channel 133 to form a droplet.

As can be seen from the above, in this embodiment, the first portion 1311 of the first channel 131 is configured to temporarily store a single first target A, and the second portion 1321 of the second channel 132 is configured to temporarily store a single second target B. A separate buffer channel 134 is configured to utilize the buffer solution to propel the single first target A and the single second target B into the oil. This obviates the need to propel the single first target A and the single second target B through the first liquid and the second liquid, so that the first channel 131 and the second channel 132 can be kept in the blocked state during the encapsulation process, to prevent other first targets and/or other second targets from entering the droplet C, thereby improving the success rate of encapsulation and reducing cell loss caused by co-encapsulation.

Based on the second embodiment, referring to FIG. 10, in some embodiments of the present disclosure, the microfluidic system further includes a waste channel 135 configured for discharging excess first liquid and second liquid, and a droplet channel 139 configured for discharging the droplet C. Both the waste channel 135 and the droplet channel 139 are in communication with the oil channel 133. In addition, the microfluidic system further includes a waste microvalve 125 corresponding to the waste channel 135 and a droplet microvalve 127 corresponding to the droplet channel 139. The waste microvalve 125 is configured for controlling opening and closing of the waste channel 135, and the droplet microvalve 127 is configured for controlling opening and closing of the droplet channel 139. When both the first microvalve 121 and the waste microvalve 125 are in the open state and the droplet microvalve 127 is in the closed state, the first liquid in the first channel 131 is allowed to flow out through the pairing channel 136, the oil channel 133, and the waste channel 135.

Based on the second embodiment, referring to FIG. 10, in some embodiments of the present disclosure, the microfluidic system further includes a waste channel 135, a droplet channel 139, a waste microvalve 125 corresponding to the waste channel 135, and a droplet microvalve 127 corresponding to the droplet channel 139. For details of the waste channel 135, the droplet channel 139, the waste microvalve 125, and the droplet channel 139, reference can be made to the description in the above embodiments. When both the second microvalve 122 and the waste microvalve 125 are in the open state and the droplet microvalve 127 is in the closed state, the second liquid in the second channel 132 is allowed to flow out through the pairing channel 136, the oil channel 133, and the waste channel 135. For the discharge of the second liquid, reference can be made to the discharge of the first liquid, and the details will not be repeated herein.

Based on the second embodiment, referring to FIG. 10, in some embodiments of the present disclosure, the microfluidic system further includes a waste channel 135, a droplet channel 139, a waste microvalve 125 corresponding to the waste channel 135, and a droplet microvalve 127 corresponding to the droplet channel 139. For details of the waste channel 135, the droplet channel 139, the waste microvalve 125, and the droplet channel 139, reference can be made to the description in the above embodiments. In this embodiment, when a droplet is formed in the oil in the oil channel 133, the waste microvalve 125 is in the closed state and the droplet microvalve 127 is in the open state, allowing the droplet to be discharged through the droplet channel 139. Based on the embodiments, switching between the discharge of the waste liquid and the discharge of the droplet can be achieved by changing the states of the waste microvalve 125 and the droplet channel 139.

Based on the second embodiment, referring to FIG. 10, in some embodiments of the present disclosure, the microfluidic system further includes a waste channel 135, a droplet channel 139, a waste microvalve 125 corresponding to the waste channel 135, and a droplet microvalve 127 corresponding to the droplet channel 139. The pairing channel 136 is in communication with an inlet end of the oil channel 133, and both the waste channel 135 and the droplet channel 139 are in communication with an outlet end of the oil channel 133. In the embodiment shown in FIG. 10, the pairing channel 136 is in communication with an upper end of the oil channel 133, and both the waste channel 135 and the droplet channel 139 are in communication with a lower end of the oil channel 133. In addition, the first channel 131 and the second channel 132 are both in communication with an inlet end of the pairing channel 136. The buffer channel 134 has two branch channels at an end thereof. The two branch channels are respectively in communication with the first portion 1311 and the second portion 1321, and the two branch channels have an identical length, so that the first target A in the first portion 1311 and the second target B in the second portion 1321 can be pushed into the pairing channel 136 substantially synchronously by the buffer solution.

Based on the second embodiment, referring to FIG. 10, in some embodiments of the present disclosure, the microfluidic system further includes an oil microvalve 123 corresponding to the oil channel 133. During discharge of the waste liquid, the oil microvalve 123 is in a closed state, allowing the waste liquid to enter the waste channel 135 through the oil channel 133. During encapsulation, the oil microvalve 123 is in an open state, allowing the oil to fill up the oil channel 133.

Based on the second embodiment, referring to FIG. 10, in some embodiments of the present disclosure, the microfluidic system further includes a detection module configured for identifying a single first target in the first channel 131. After the detection module identifies the single first target, the first microvalve 121 is in the closed state to keep the single first target in the first portion 1311. It should be noted that in this embodiment, the first microvalve 121 being in the closed state means switching from the open state to the closed state.

In some other embodiments, the detection module is configured for identifying a single second target in the second channel 132. After the detection module identifies a single second target, the second microvalve 122 is in the closed state to keep the single second target in the second portion 1321. It should be noted that in this embodiment, the second microvalve 122 being in the closed state means switching from the open state to the closed state.

In some embodiments of the present disclosure, the microfluidic system further includes a waste microvalve 125 corresponding to the waste channel 135 configured for controlling opening and closing of the waste channel 135 when the detection module is detecting a single first target A. Before the detection module identifies the single first target A, the droplet microvalve 127 is in the closed state and both the first microvalve 121 and the waste microvalve 125 are in the open state, allowing the first liquid in the first channel 131 to flow out through the pairing channel 136, the oil channel 133, and the waste channel 135. It should be noted that in this embodiment, the droplet microvalve 127 being in the closed state means remaining in the closed state, and both the first microvalve 121 and the waste microvalve 125 being in the open state means remaining in the open state.

In some embodiments of the present disclosure, before the detection module identifies the single second target B, the droplet microvalve 127 is in the closed state and both the second microvalve 122 and the waste microvalve 125 are in the open state, allowing the second liquid in the second channel 132 to flow out through the pairing channel 136, the oil channel 133, and the waste channel 135 when the detection module is detecting the single second target B. It should be noted that in this embodiment, the droplet microvalve 127 being in the closed state means remaining in the closed state, and the second microvalve 122 and the waste microvalve 125 being in the open state means remaining in the open state.

For different detection methods of the detection module, reference can be made to the description of the visual detection, fluorescence detection, and electrode-based detection in the first embodiment, and the details will not be repeated herein.

A third embodiment of the present disclosure also provides a pairing method, including following steps.

At a step of S100, a single first target is identified from a first liquid carrying the first target, and upon identification of the single first target, the first liquid is cut off to stop the single first target in a target region. In some embodiments, this step may be executed based on the microfluidic system of the first embodiment or the second embodiment, i.e., the target region in this step may be the pairing channel 136 in the first embodiment or the first portion 1311 in the second embodiment.

At a step of S200, a single second target is identified from a second liquid carrying the second targets, and upon identification of the single second target, the second liquid is cut off to stop the second first target in the target region. In some embodiments, this step may be executed based on the microfluidic system of the first embodiment or the second embodiment, i.e., the target region in this step may be the pairing channel 136 in the first embodiment or the second portion 1321 in the second embodiment.

At a step of S300, after the single first target and the single second target both stay in the target region and both the first liquid and the second liquid are in a cut-off state, both the single first target and the single second target in the target region are pushed into oil by a third liquid to form a droplet.

It should be noted that the order in which S100 and S200 are executed is not limited herein.

Based on the third embodiment, in some embodiments of the present disclosure, a method of identifying the single first target from the first liquid carrying first targets includes: acquiring an image of a detection region, and identifying the single first target based on image information in the image, where the first liquid is allowed to enter the target region after passing through the detection region.

Based on the third embodiment, in some embodiments of the present disclosure, a method of identifying the single second target from the second liquid carrying second targets includes: acquiring an image of a detection region, and identifying the single second target based on image information in the image, where the second liquid is allowed to enter the target region after passing through the detection region.

For details of these embodiments, reference can be made to the description of the visual detection scheme in the first embodiment.

Based on the third embodiment, in some embodiments of the present disclosure, a method of identifying the single first target from the first liquid carrying first targets includes: detecting an optical signal in a detection region, and identifying the single first target based on the detected optical signal, where the first liquid is allowed to enter the target region after passing through the detection region. The optical signal may be fluorescence emitted by the first target after being excited.

Based on the third embodiment, in some embodiments of the present disclosure, a method of identifying the single second target from the second liquid carrying second targets includes: detecting an optical signal in a detection region, and identifying the single second target based on the detected optical signal, where the second liquid is allowed to enter the target region after passing through the detection region. The optical signal may be fluorescence emitted by the second target after being excited.

For details of these embodiments, reference can be made to the description of the fluorescence detection scheme in the first embodiment.

Based on the third embodiment, in some embodiments of the present disclosure, a method of identifying the single first target from the first liquid carrying first targets includes: detecting an electrical signal generated by an electrode arranged in a detection region, and identifying the single first target based on the detected electrical signal, where the first liquid is allowed to enter the target region after passing through the detection region.

Based on the third embodiment, in some embodiments of the present disclosure, a method of identifying the single second target from the second liquid carrying second targets includes: detecting an electrical signal generated by an electrode arranged in the detection region, and identifying the single second target based on the detected electrical signal, where the second liquid is allowed to enter the target region after passing through the detection region. In this embodiment, the signal detected by the electrode may be a change in amplitude and phase difference.

In the above embodiments, the signal detected by the electrode may be a change in amplitude and phase difference.

For details of these embodiments, reference can be made to the description of the electrode-based detection scheme in the first embodiment.

In the above embodiments, in a flow direction of the first liquid, the detection region is located upstream of the target region. For example, when the target region is the pairing channel 136 in the first embodiment, the detection region may be the first portion 1311 of the first channel 131 in communication with the pairing channel 136. When the target region is the first portion 1311 in the second embodiment, the detection region may be another region in the first channel 131.

In a flow direction of the second liquid, the detection region is located upstream of the target region. For example, when the target region is the pairing channel 136 in the first embodiment, the detection region may be the second portion 1321 of the second channel 132 in communication with the pairing channel 136. When the target region is the second portion 1321 in the second embodiment, the detection region may be another region in the second channel 132.

Based on the third embodiment, in some embodiments of the present disclosure, the pairing method further includes: pushing the droplet containing the single first target and the single second target to pass through a fusion region, and applying an electric field to the fusion region to incorporate a fourth liquid into the droplet. In this embodiment, the electric field may be applied using a fusion electrode assembly.

The embodiments of the present disclosure have been described in detail above with reference to the accompanying drawings, but the present disclosure is not limited to the above embodiments, and various changes may be made within the knowledge of those having ordinary skills in the art without departing from the spirit of the present disclosure. In addition, the embodiments of the present disclosure and the features in the embodiments may be combined with each other without conflict.

## Claims

1. A microfluidic system for target object pairing, comprising a first channel, a second channel, an oil channel, a buffer channel, and a pairing channel, wherein the pairing channel is in communication with the oil channel, and the first channel, the second channel, and the buffer channel are all in communication with the pairing channel;
wherein the microfluidic system further comprises a pairing microvalve corresponding to the pairing channel, a first microvalve corresponding to the first channel, a second microvalve corresponding to the second channel, and a buffer microvalve corresponding to the buffer channel, wherein the pairing microvalve is configured to control opening and closing of the pairing channel, the first microvalve is configured to control opening and closing of the first channel, the second microvalve is configured to control opening and closing of the second channel, and the buffer microvalve is configured to control opening and closing of the buffer channel;
wherein both the first microvalve and the second microvalve are in a closed state, and both the pairing microvalve and the buffer microvalve are in an open state in response to a single first target and a single second target being present in the pairing channel, so that a buffer solution in the buffer channel pushes both the single first target and the single second target in the pairing channel into oil phase in the oil channel to form a droplet.

2. The microfluidic system for target object pairing of claim 1, further comprising a waste channel in communication with the pairing channel and a waste microvalve corresponding to the waste channel, wherein the waste microvalve is configured to control opening and closing of the waste channel;
wherein in response to the pairing microvalve being in the closed state and both the first microvalve and the waste microvalve being in the open state, liquid in the first channel flows out through the pairing channel and the waste channel; and/or
in response to the pairing microvalve being in the closed state and both the second microvalve and the waste microvalve being in the open state, liquid in the second channel flows out through the pairing channel and the waste channel.

3. The microfluidic system for target object pairing of claim 2, wherein the first channel, the second channel, the buffer channel, and the waste channel are all located on a side of the pairing microvalve away from the oil channel.

4. The microfluidic system for target object pairing of claim 2, wherein the first channel and the second channel are located on one side of the pairing channel, and the waste channel is located on another side of the pairing channel.

5. The microfluidic system for target object pairing of claim 2, wherein the microfluidic system further comprises at least one of:
at least a first portion of the first channel which is in communication with the pairing channel intersects the pairing channel;
at least a second portion of the second channel which is in communication with the pairing channel intersects the pairing channel; and
at least a third portion of the waste channel which is in communication with the pairing channel intersects the pairing channel.

6. The microfluidic system for target object pairing of claim 2, wherein at least a fourth portion of the buffer channel which is in communication with the pairing channel is arranged coaxially with the pairing channel.

7. The microfluidic system for target object pairing of claim 2, wherein the waste channel is located between the first channel and the second channel in an extending direction of the pairing channel.

8. The microfluidic system for target object pairing of claim 1, wherein the microfluidic system further comprises a detection module configured to identify the single first target in the first channel, wherein after the detection module identifies the single first target, both the first microvalve and the pairing microvalve are in the closed state to keep the single first target in the pairing channel; and/or
the microfluidic system further comprises a detection module configured to identify the single second target in the second channel, wherein after the detection module identifies the single second target, both the second microvalve and the pairing microvalve are in the closed state to keep the single second target in the pairing channel.

9. The microfluidic system for target object pairing of claim 8, further comprising a waste channel in communication with the pairing channel and a waste microvalve corresponding to the waste channel, wherein the waste microvalve is configured to control opening and closing of the waste channel;
wherein before the detection module identifies the single first target, the pairing microvalve is in the closed state and both the first microvalve and the waste microvalve are in the open state, so that liquid in the first channel flows out through the pairing channel and the waste channel; and/or
before the detection module identifies the single second target, the pairing microvalve is in the closed state and both the second microvalve and the waste microvalve are in the open state, so that the liquid in the second channel flows out through the pairing channel and the waste channel.

10. The microfluidic system for target object pairing of claim 8, wherein the detection module comprises a camera and a controller configured to control the camera to capture an image;
wherein the image comprises at least a first image of a first portion of the first channel which is in communication with the pairing channel, and the controller is further configured to identify the single first target based on the first image; and/or
the image comprises at least a second image of a second portion of the second channel which is in communication with the pairing channel, and the controller is further configured to identify the single second target based on the second image.

11. The microfluidic system for target object pairing of claim 10, wherein the image comprises at least a third image of the pairing channel, and the controller is configured to identify whether the single first target and/or the single second target is present in the pairing channel based on the third image.

12. The microfluidic system for target object pairing of claim 8, wherein the detection module comprises a first light source, a first light detection device, and a controller configured to control the first light source to emit first detection light to the first channel, so that the first detection light is configured to excite the single first target to emit first fluorescence upon irradiating the single first target, and the first light detection device detects presence of the single first target in response to the first light detection device detecting the first fluorescence; and/or
the detection module comprises a second light source, a second light detection device, and a controller configured to control the second light source to emit second detection light to the second channel, wherein the second detection light is configured to excite the single second target to emit second fluorescence upon irradiating the single second target, and the controller identifies presence of the single second target in response to the second light detection device detecting the second fluorescence.

13. The microfluidic system for target object pairing of claim 8, wherein the detection module comprises a first detection electrode assembly extending into the first channel, and a controller configured to identify the single first target based on a signal detected by the first detection electrode assembly; and/or
the detection module comprises a second detection electrode assembly extending into the second channel, and a controller configured to identify the single second target based on a signal detected by the second detection electrode assembly.

14. The microfluidic system for target object pairing of claim 8, wherein after the detection module identifies the single first target, the first microvalve is switched from the open state to the closed state after a time delay during which the single first target enters the pairing channel from the first channel; and/or
after the detection module identifies the single second target, the second microvalve is switched from the open state to the closed state after a time delay during which the single second target enters the pairing channel from the second channel.

15. The microfluidic system for target object pairing of claim 1, further comprising an injection channel in communication with the oil channel, wherein the pairing channel has a first communication portion which is in communication with the oil channel, and a second communication portion which is in communication with the oil channel, and located downstream of the first communication portion in a flow direction of the oil in the oil channel; and
wherein the microfluidic system further comprises a fusion electrode assembly configured to apply an electric field to the second communication portion to incorporate liquid in the injection channel into the droplet that passes through the second communication portion.

16. The microfluidic system for target object pairing of claim 15, further comprising a processing region in which the first channel, the second channel, the oil channel, the buffer channel, the pairing channel, and the injection channel are all located; and
wherein the fusion electrode assembly comprises a first electrode surrounding the processing region and a second electrode.

17. The microfluidic system for target object pairing of claim 16, wherein two ends of the first electrode are parallel to the second electrode, and the second electrode is between the two ends of the first electrode.

18. The microfluidic system for target object pairing of claim 1, further comprising an oil microvalve corresponding to the oil channel, wherein the oil microvalve is configured to control opening and closing of the oil channel.

19. The microfluidic system for target object pairing of claim 1, further comprising a first microvalve control channel, which comprises a membrane between the first channel and the first microvalve control channel, wherein the membrane is configured to be driven to protrude toward the first channel to enter a closed state; and
wherein an inner wall of the first channel is an arc-shaped inner wall.

20. The microfluidic system for target object pairing of claim 1, further comprising a microfluidic chip which comprises a base layer, a control layer, and a channel layer stacked in sequence;
wherein the first channel, the second channel, the oil channel, the buffer channel, and the pairing channel are arranged in the channel layer; and
wherein the pairing microvalve, the first microvalve, the second microvalve, and the buffer microvalve are arranged in the control layer.

21. The microfluidic system for target object pairing of claim 1, wherein the first target is a cell, and the second target is a microsphere.

22. The microfluidic system for target object pairing of claim 21, wherein the microsphere is a magnetic microsphere.

23. The microfluidic system for target object pairing of claim 23, wherein the microsphere is a fluorescent magnetic microsphere.

24. The microfluidic system for target object pairing of claim 1, wherein the first target is a first cell, and the second target is a second cell.

25. A microfluidic system for target object pairing, comprising a first channel, a second channel, an oil channel, a buffer channel, and a pairing channel, wherein the pairing channel is in communication with the oil channel, the first channel and the second channel are both in communication with the pairing channel, the buffer channel is respectively in communication with the first channel and the second channel, the first channel comprises a first portion in communication with the pairing channel, and the second channel comprises a second portion in communication with the pairing channel;
wherein the microfluidic system further comprises a first microvalve corresponding to the first channel, a second microvalve corresponding to the second channel, and a buffer microvalve corresponding to the buffer channel, wherein the first microvalve is configured to control opening and closing of the first channel, the second microvalve is configured to control opening and closing of the second channel, and the buffer microvalve is configured to control opening and closing of the buffer channel;
wherein the first microvalve and the second microvalve are in a closed state, and the buffer microvalve is in an open state in response to a single first target being present in the first portion and a single second target being present in the second portion, so that a buffer solution in the buffer channel pushes the single first target in the first portion and the single second target in the second portion into an oil phase in the oil channel through the pairing channel to form a droplet.

26. The microfluidic system for target object pairing of claim 25, further comprising a waste channel, a droplet channel, a waste microvalve corresponding to the waste channel, and a droplet microvalve corresponding to the droplet channel, wherein the waste channel and the droplet channel are respectively in communication with the oil channel, the waste microvalve is configured to control opening and closing of the waste channel, and the droplet microvalve is configured to control opening and closing of the droplet channel; and
wherein liquid in the first channel flows out through the pairing channel, the oil channel, and the waste channel in response to both the first microvalve and the waste microvalve being in the open state and the droplet microvalve being in the closed state; and/or
wherein liquid in the second channel flows out through the pairing channel, the oil channel, and the waste channel in response to both the second microvalve and the waste microvalve being in the open state and the droplet microvalve being in the closed state.

27. The microfluidic system for target object pairing of claim 25, further comprising a waste channel, a droplet channel, a waste microvalve corresponding to the waste channel, and a droplet microvalve corresponding to the droplet channel, wherein the waste channel and the droplet channel are respectively in communication with the oil channel, the waste microvalve is configured to control opening and closing of the waste channel, and the droplet microvalve is configured to control opening and closing of the droplet channel;
wherein the waste microvalve is in the closed state and the droplet microvalve is in the open state in response to a droplet being formed in the oil in the oil channel, so that the droplet is discharged through the droplet channel.

28. The microfluidic system for target object pairing of claim 25, further comprising a waste channel, a droplet channel, a waste microvalve corresponding to the waste channel, and a droplet microvalve corresponding to the droplet channel, wherein the waste microvalve is configured to control opening and closing of the waste channel, and the droplet microvalve is configured to control opening and closing of the droplet channel;
wherein the pairing channel is in communication with an inlet end of the oil channel, and the waste channel and the droplet channel are both in communication with an outlet end of the oil channel.

29. The microfluidic system for target object pairing of claim 25, further comprising a detection module configured to identify the single first target in the first channel, wherein after the detection module identifies a single first target, the first microvalve is in the closed state to keep the single first target in the first portion; and/or
wherein the microfluidic system further comprises a detection module configured to identify the single second target in the second channel, wherein after the detection module identifies a single second target, the second microvalve is in the closed state to keep the single second target in the second portion.

30. A pairing method, comprising:
identifying a single first target from first liquid carrying first targets, and upon identification of the single first target, cutting off the first liquid to stop the single first target in a target region;
identifying a single second target from second liquid carrying second targets, and upon identification of the single second target, cutting off the second liquid to stop the single second target in the target region;
after the single first target and the single second target both stay in the target region, pushing the single first target and the single second target in the target region into an oil by a third liquid to form a droplet.

31. The pairing method of claim 30, wherein identifying a single first target from first liquid carrying first targets comprises: acquiring an image of a detection region, and identifying the single first target based on image information in the image, and the first liquid enters the target region after passing through the detection region; and/or
wherein identifying a single second target from second liquid carrying second targets comprises:
acquiring an image of a detection region, and identifying the single second target based on image information in the image, and the second liquid enters the target region after passing through the detection region.

32. The pairing method of claim 30, wherein identifying a single first target from first liquid carrying first targets comprises: detecting an optical signal in a detection region, and identifying the single first target based on the detected optical signal, and the first liquid enters the target region after passing through the detection region; and/or
wherein identifying a single second target from second liquid carrying second targets comprises:
detecting an optical signal in a detection region, and identifying the single second target based on the detected optical signal, and the second liquid enters the target region after passing through the detection region.

33. The pairing method of claim 30, wherein identifying a single first target from first liquid carrying first targets comprises: detecting an electrical signal generated by an electrode in a detection region, and identifying the single first target based on the detected electrical signal, and the first liquid enters the target region after passing through the detection region; and/or
wherein identifying a single second target from second liquid carrying second targets comprises:
detecting an electrical signal generated by an electrode in the detection region, and identifying the single second target based on the detected electrical signal, and the second liquid is allowed to enter the target region after passing through the detection region.

34. The pairing method of claim 30, further comprising: pushing the droplet containing the single first target and the single second target to pass through a fusion region, and applying an electric field to the fusion region to incorporate a fourth liquid into the droplet.
